# FASCICULE DE BREVET EUROPEEN

(11) **EP 2 531 517 B1**
(45) Date de publication et mention de la délivrance du brevet: **31.05.2017**
(21) Numéro de dépôt: 11708524.1
(22) Date de dépôt: 01.02.2011
(51) Int. Cl.: C07K 7/06, C07K 7/08, A61K 38/04, A61K 8/64, A61Q 17/00, A61Q 19/08, A61K 38/08, A61K 38/10, A61P 17/00

(54) **PEPTIDES ACTIVATEURS DE LA CASPASE-14 ET COMPOSITIONS LES COMPRENANT**
CASPASE-14 AKTIVIERENDE PEPTIDE UND ZUSAMMENSETZUNGEN DIESE ENTHALTEND
CASPASE-14 ACTIVATOR PEPTIDES AND COMPOSITIONS COMPRISING SAID PEPTIDES

(30) Priorité: 05.02.2010 FR 1000463
(43) Date de publication de la demande: 12.12.2012
(73) Titulaire: ISP Investments Inc., Wilmington, DE 19805 (US)
(72) Inventeur: DAL FARRA, Claude, Kerhonkson New York 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2011/000060
(87) Numéro de publication internationale: WO 2011/095705

(56) Documents cités:
- WO-A1-98/43621
- WO-A2-2006/124477

## Description

La présente invention se situe dans les domaines de la cosmétique et de la pharmacie. Elle se rapporte à des composés peptidiques de formule générale (I) suivante :

R₁ - (AA)ₙ - X₁ - X₂ - Ile - Gln - Ala - Cys - Arg - Gly - X₃ - (AA)ₚ - R₂

en tant qu'activateurs de la caspase-14, ainsi que leurs utilisations en cosmétique et/ou pharmaceutique afin de prévenir et/ou réparer les dommages subis par l'ADN, prévenir et/ou traiter les signes cutanés du vieillissement et du photo-vieillissement et améliorer la fonction barrière de la peau.

La principale fonction de la peau, qui est l'organe le plus grand du corps humain, est de protéger l'organisme contre un grand nombre d'agressions, notamment extérieures. A titre d'exemple, on peut citer des agressions telles que la pollution, les UV, ou encore les produits à caractère irritant tels que les tensioactifs, les conservateurs ou les parfums, les agressions mécaniques, telles que les abrasions, le rasage ou l'épilation. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. La sécheresse de l'atmosphère est également une cause importante d'agression cutanée. Ces agressions extérieures aboutissent à une altération de la fonction barrière de la peau, qui se traduit par un inconfort cutané, des phénomènes sensoriels désagréables, tels que des tiraillements ou des démangeaisons, voire des fragilités excessives et des rougeurs. Par ailleurs, ces agressions extérieures participent à l'accélération du vieillissement dit « extrinsèque » de la peau. En effet, le vieillissement extrinsèque, est dû à des facteurs environnementaux auxquels est soumis l'organisme tout au long de sa vie, facteurs l'on regroupe sous le terme d'agressions extérieures.

Outre son rôle de barrière physique, la peau a un rôle de barrière imperméable à l'eau et ce afin d'éviter la déshydratation. Cette barrière que représente la peau, est constituée par plusieurs couches composées de cellules de natures diverses qui permettent d'assurer à la peau son renouvellement continuel. Ce processus de renouvellement passe avant tout par un phénomène de desquamation des cellules qui se trouvent à la surface de la peau, phénomène qui doit être compensé par le renouvellement de l'épiderme assuré par les kératinocytes de la couche basale qui se divisent activement et se différencient en cellules de la couche cornée ou cornéocytes. Ces activités de renouvellement, mais aussi de réparation de la peau dans le cas de dommages tels que les rayonnements UV, sont extrêmement contrôlées par un ensemble de voies de signalisation. Parmi ces voies de signalisation, l'une d'entre elles fait intervenir une protéase, la caspase-14. La caspase-14 est un membre unique au sein de la famille des caspases. En effet, contrairement aux autres membres de la famille des caspases qui sont exprimés de façon ubiquitaire, caspase-14 est uniquement exprimée et active dans l'épiderme et est absente de la plupart des autres tissus adultes (Eckhart et al., J. Invest. Dermatol., 2003, 44:1148-1151). Récemment, il a été prouvé le rôle crucial de la caspase-14 dans la formation de la barrière que forme l'épiderme (Denecker et al., Nat. Cell. Biol. 2007, 9 :666-674). En effet, celle-ci est exprimée et exerce son activité protéolytique seulement dans les couches de l'épiderme où ont lieu la différenciation et la « cornification », ainsi que dans le follicule pileux (Lippens et al., Cell Death Differ., 2000, 10 :257-259). Or, il a été prouvé que la caspase-14 jouait un grand rôle en particulier dans les processus de maintien de l'hydratation, de formation des cornéocytes, et de protection contre l'apoptose, notamment lors d'agressions extérieures comme celles dues aux rayonnements UV (Denecker et al., J. Cell Biology, 2008, 451-458). Par ailleurs, il a été démontré que la caspase-14 était responsable du clivage de la profilaggrine en filaggrine, filaggrine qui est ensuite hydrolysée en peptides et acides aminés qui forment le Natural Moisturizing Factor (Hoste et al., J. Invest. Dermatol. Abstract, 2007, S71). L'ensemble de ces conclusions ont, entre autre, conduit à l'élaboration de compositions pharmaceutiques comprenant la caspase-14 en tant qu'agent actif, compositions utilisables en tant qu'écran solaire (WO 2008025830). Aussi des peptides comprenant la séquence IQACRG avec différentes modifications N- ou C-terminales ont été décrits (WO 98/43621 et WO 2006/124477). Cependant ils sont proposés à des fins de traitements pharmaceutiques, inhibiteurs de caspase, afin de traiter des conditions liées à l'apoptose. Cependant, il n'existe aucun composé cosmétique à l'heure actuelle, permettant d'activer la caspase-14 et par là même, la conversion de la profilaggrine en filaggrine, pour protéger la peau contre les rayonnements UV, et améliorer la fonction barrière, alors que le besoin de soin innovant de ce type existe.

Aujourd'hui la Demanderesse a mise en évidence que des composés peptidiques de formule générale (I) suivante :

R₁ - (AA)ₙ - X₁ - X₂ - Ile - Gln - Ala - Cys - Arg - Gly - X₃ - (AA)ₚ - R₂

étaient de très bons agents activateurs de la caspase-14, et avaient une action significative sur l'amélioration de la fonction barrière de la peau, ainsi que sur la protection de la peau contre les agressions extérieures telles que les rayonnements UV, grâce notamment, à une fonction de protection de l'ADN.

Les composés peptidiques selon l'invention se caractérisent par le fait qu'ils :
- activent la caspase-14 et par conséquent ils activent son action protéolytique vis-à-vis de la profilaggrine ;
- permettent de conserver l'hydratation de l'épiderme ;
- préviennent et réparent les dommages de l'ADN des cellules de la peau soumises à des rayonnements UVB et ;
- optimisent le rôle de barrière de l'épiderme

La présente invention a donc pour premier objet un composé peptidique de formule générale suivante (I) :

R₁ - (AA)ₙ - X₁ - X₂ - Ile - Gln - Ala - Cys - Arg - Gly - X₃ - (AA)ₚ - R₂.

La présente invention a pour second objet une composition cosmétique comprenant à titre de principe actif ledit composé peptidique de formule (I).

En outre, la présente invention a pour troisième objet l'utilisation d'une composition cosmétique comprenant ledit composé peptidique de formule (I) pour (i) pour protéger et/ou réparer les dommages subis par l'ADN, activer la caspase-14 ainsi que la formation de filaggrine, (ii) pour prévenir et/ou traiter les signes cutanés du vieillissement et du photo-vieillissement et, (iii) améliorer la fonction barrière de la peau ainsi que l'hydratation de l'épiderme.

Enfin, la présente invention a pour quatrième objet un procédé de traitement cosmétique de la peau ou des phanères à traiter, à l'aide de la composition comprenant ledit composé peptidique de formule(I).

Le premier objet de la présente invention se rapporte à un composé peptidique de formule générale (I) :

R₁ - (AA)ₙ - X₁ - X₂ - Ile - Gln - Ala - Cys - Arg - Gly - X₃ - (AA)ₚ - R₂ (I)

Dans laquelle,
X₁ représente un acide aspartique, un acide glutamique ou aucun acide aminé,
X₂ représente une asparagine, une proline, une sérine ou aucun acide aminé,
X₃ représente une asparagine, une arginine, une leucine ou aucun acide aminé,
AA représente un acide aminé quelconque, et n et p sont des nombres entiers compris entre 0 et 2,
R₁ représente la fonction amine primaire de l'acide aminé N-terminal, libre, R₂ représente le groupe hydroxyle de la fonction carboxyle de l'acide aminé C-terminal, substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 6 à 13 résidus d'acides aminés.

Le terme « composé peptidique » ou « peptide » désigne un enchaînement de deux ou plusieurs acides aminés liés entre eux par des liaisons peptidiques ou par des liaisons peptidiques modifiées.

Par «composé peptidique» ou «peptide», il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

Les acides aminés constituant le composé peptidique selon l'invention peuvent être sous configuration Lévogyre c'est-à-dire L- et/ou Dextrogyre c'est-à-dire D-. Le peptide selon l'invention peut donc être sous forme L-, D- ou DL-.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans les domaines de la cosmétique et de la pharmacie. De préférence, on utilise pour protéger la fonction amine primaire de l'acide aminé N-terminal, une substitution par un groupement R₁ de type acyle possédant une chaîne alkyle de C₁ à C₃₀ saturée ou insaturée, pouvant être choisie parmi un groupement acétyle ou un groupement aromatique. De préférence, on utilise pour protéger la fonction carboxyle de l'acide aminé C-terminal, une substitution par un groupement R₂ de type chaîne alkyle de C₁ à C₃₀, ou un groupement NH₂, NHY ou NYY avec Y représentant une chaîne alkyle de C₁ à C₄.

Le peptide selon l'invention peut être protégé au niveau de l'extrémité N-terminale, C-terminale ou au niveau des deux extrémités.

Dans un premier mode de réalisation de l'invention, dans la formule générale (I),
X₁ représente un acide aspartique, un acide glutamique ou aucun acide aminé,
X₂ représente une asparagine, une proline, une sérine ou aucun acide aminé,
X₃ représente une asparagine, une arginine, une leucine ou aucun acide aminé,
les nombres entiers n et p sont égaux à zéro
R₁ représente la fonction amine primaire de l'acide aminé N-terminal, libre, R₂ représente le groupe hydroxyle de la fonction carboxyle de l'acide aminé C-terminal, substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 6 à 9 résidus d'acides aminés.

Dans un second mode de réalisation préféré, le composé peptidique correspond à une des formules suivantes :
(SEQ ID n°1) Asp - Pro - Ile - Gln - Ala - Cys - Arg - Gly - NH₂
(SEQ ID n°2) Ile - Gln - Ala - Cys - Arg - Gly - NH₂
(SEQ ID n°3) Asn - Arg - Ile - Gln - Ala - Cys - Arg - Gly- NH₂
(SEQ ID n°4) Pro - Ile - Gln - Ala - Cys - Arg - Gly - Phe - NH₂

Le peptide de formule générale (I) selon l'invention peut être obtenu soit par synthèse chimique classique (en phase solide ou en phase homogène liquide), soit par synthèse enzymatique (Kullmann et al., J. Biol. Chem. 1980, 225, 8234), à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Enfin, le principe actif peut être un peptide unique, un mélange de peptides ou de dérivés peptidiques et/ou constitué de dérivés d'acides aminés.

Le composé peptidique selon l'invention peut être utilisé à titre de médicament.

Selon un mode de réalisation avantageux de l'invention, le composé peptidique selon l'invention est solubilisé dans un ou plusieurs solvants physiologiquement adaptés, classiquement utilisés par l'homme du métier, comme l'eau, le glycérol, l'éthanol, le propanediol, le butylène glycol, le dipropylène glycol, les diglycols éthoxylés ou propoxylés, les polyols cycliques, ou tout mélange de ces solvants.

Selon encore un autre mode de réalisation avantageux de l'invention, le composé peptidique selon l'invention est solubilisé dans un vecteur cosmétique ou pharmaceutique comme les liposomes, ou adsorbé sur des polymères organiques poudreux, des supports minéraux comme les talcs et bentonites, et plus généralement solubilisé dans, ou fixé sur, tout vecteur physiologiquement adapté.

Le second objet de la présente invention se rapporte à une composition cosmétique comprenant à titre de principe actif ledit composé peptidique de formule générale (I). De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Les compositions destinées à être appliquées sur la peau peuvent se présenter sous forme de crème, émulsion huile-dans-eau, ou eau-dans-huile ou émulsion multiple, solution, suspension, microémulsion, gel aqueux ou anhydre, sérum, ou encore de dispersion de vésicules, de patch, de spray, d'onguent, de pommade, de lotion, de colloïde, de lait, de lotion, de stick ou encore de poudre, tous adaptes à une application sur la peau, les lèvres et/ou les phanères.

Préférentiellement, ledit composé peptidique est présent dans la composition à une concentration comprise entre 0,0005 et 500 ppm environ, et préférentiellement à une concentration comprise entre 0,01 et 5 ppm.

Encore plus préférentiellement, la composition selon l'invention contient en outre, au moins un autre principe actif favorisant l'action dudit composé peptidique. On peut citer, de manière non limitative, les classes d'ingrédients suivantes : d'autres agents actifs peptidiques, des extraits de végétaux, des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, des agents modulants la différenciation, la pigmentation ou la dépigmentation cutanée, des agents stimulants la pousse des ongles ou des cheveux etc. Préférentiellement, on utilisera un agent présentant une activité dans le domaine des antirides, tel qu'un agent anti-radicalaire ou antioxydant, ou un agent stimulant la synthèse de macromolécules dermiques, ou encore un agent stimulant le métabolisme énergétique. Plus particulièrement, le principe actif est choisi parmi les vitamines, les phytostérols, les flavonoïdes, la DHEA et/ou un de ses précurseurs ou un de ses dérivés chimiques ou biologiques, un inhibiteur de métalloprotéinase, ou un rétinoïde. En outre, des additifs tels que des solvants, des diluants, des colorants, des filtres solaires, des agents auto-bronzants, des pigments, des charges, des conservateurs, des absorbeurs d'odeurs, des agents épaississants, émulsifiants, humectants, émollients, des parfums, des antioxydants, des agents filmogènes, chélatants, séquestrants, conditionnants...peuvent être ajoutés à la composition.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être compris entre 0,01 et 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids, par rapport au poids total de la composition.

Un troisième objet de l'invention se rapporte à l'utilisation d'une composition comprenant un composé peptidique selon l'invention pour prévenir et/ou réparer les dommages subis par l'ADN, et activer la caspase-14 ainsi que la formation de filaggrine. On entend par composé peptidique destiné à « prévenir et/ou réparer les dommages subis par l'ADN » un composé ou dérivé peptidique biologiquement actif capable de corriger les dommages dus à des réactions photochimiques entre les bases de l'ADN, comme par exemple la formation de dimères pyrimidiques de cyclobutane. On entend par composé peptidique qui «permet d'activer la caspase-14», tout peptide ou dérivé biologiquement actif capable d'augmenter la quantité de caspase-14, soit par augmentation de la synthèse protéique de celle-ci (par modulation directe ou indirecte de l'expression génique), soit par d'autres processus biologiques tels que la stabilisation ou non des transcrits d'ARN messager. On entend par composé peptidique qui permet d'augmenter « la formation de filaggrine », tout peptide ou dérivé biologiquement actif capable d'augmenter la quantité de profilaggrine convertie en filaggrine, par l'intermédiaire de l'augmentation de l'activité protéolytique de la caspase-14 ou par augmentation de la quantité de caspase-14.
En particulier, la composition selon l'invention sera utilisée pour prévenir et/ou réparer les dommages subis par l'ADN lors d'agressions extérieures. Par « agressions extérieures », on entend les agressions que peut produire l'environnement. A titre d'exemple, on peut citer des agressions telles que la pollution, les rayonnements UV, ou encore les produits à caractère irritant tels que les tensioactifs, les conservateurs ou les parfums, les agressions mécaniques, telles que les abrasions, le rasage ou l'épilation. Cependant, préférentiellement, les agressions extérieures sont principalement constituées par les rayonnements UV, et en particulier les UVB.

Un autre objet de l'invention concerne l'utilisation d'une composition cosmétique comprenant ledit composé peptidique et un milieu cosmétiquement acceptable pour prévenir et/ou traiter les signes cutanés du vieillissement et du photo-vieillissement. Les « signes cutanés du vieillissement » incluent, mais ne se limitent pas à, toutes les manifestations visibles sur la peau causées par le vieillissement. On entend en particulier les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique. En outre, on entend aussi par « signes cutanés du vieillissement », les pores élargis, les imperfections, la décoloration, les taches de vieillesse, les kératoses, la perte de collagène, et autres changements du derme et de l'épiderme, mais également toutes modifications de l'aspect extérieur de la peau et des phanères dues au vieillissement comme, par exemple, les rugosités superficielles de la couche cornée, mais également toute modification interne de la peau qui ne se traduit pas systématiquement par un aspect extérieur modifié comme, par exemple, l'amincissement du derme. Par « photo-vieillissement » on entend le vieillissement prématuré de la peau causé par l'exposition prolongée et cumulative au soleil.

Préférentiellement, l'invention porte sur l'utilisation d'une composition telle que décrite précédemment pour améliorer la fonction barrière de la peau ainsi que l'hydratation de l'épiderme.

Enfin, un dernier objet de la présente invention se rapporte à un procédé de traitement cosmétique caractérisé en ce que l'on applique topiquement sur la peau ou les phanères à traiter, une composition comprenant une quantité efficace de composé peptidique selon l'invention pour prévenir et/ou traiter les signes cutanés du vieillissement et du photo-vieillissement, et prévenir et/ou réparer les dommages dus aux rayonnements UV. Dans un mode de réalisation particulier, la composition est appliquée avant une exposition de la peau au soleil, et constitue donc un excellent soin avant-soleil permettant de prévenir les dommages au niveau de l'ADN.

Dans un second mode de réalisation de l'invention, la composition est appliquée sur la peau en tant que soin après-soleil afin de réparer les éventuels dommages subis par la peau au niveau de l'ADN.
Dans un dernier mode préférentiel de réalisation de l'invention, la composition est appliquée soit le matin en tant que soin anti-âge de jour, soit le soir au coucher en tant que soin réparateur de nuit. En cas d'application en soin de jour, la composition permettra une protection de la peau vis-à-vis des agressions de l'environnement comme les rayonnements UVB, en limitant l'apparition de dommages au niveau de l'ADN. En tant que soin de nuit, la composition jouera un rôle de réparation des éventuels dommages que la peau aura subie lors de la journée.

Dans les traitements de dommages au niveau de l'ADN par les rayonnements UV, la présente invention se rapporte à des compositions aux fins thérapeutiques. Dans les autres cas, une utilisation non-thérapeutique cosmétique est visée.

Les exemples suivants décrivent et démontrent l'efficacité de composés peptidiques tels que décrits selon l'invention.

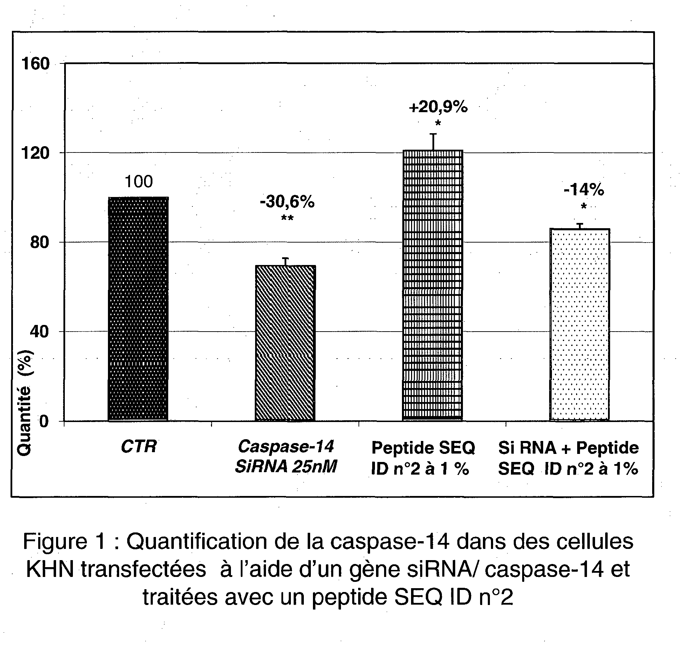

La Figure 1 est un histogramme présentant les résultats d'un immunotransfert réalisé avec des kératinocytes humains normaux (KHN) transfectés à l'aide d'un gène siRNA/ caspase-14.

Les Figure 2a et 2b sont des histogrammes présentant les résultats de 2 tests comètes réalisés sur des kératinocytes humains normaux (KHN) soumis à des rayonnements UV.

### Exemple 1 : Etude de l'expression de la caspase-14 dans des kératinocytes humains normaux, en présence du peptide SEQ ID n° 2

Le but de cette étude est de déterminer l'expression de la caspase-14 dans des kératinocytes humains normaux traités ou non à l'aide d'un peptide selon l'invention.

### Protocole :

Des kératinocytes humains normaux, KHN, en culture sont traités avec le peptide SEQ ID n°2 à 1 % ou à 3 % pendant 24 heures. Les cellules sont ensuite lavées, fixées au paraformaldéhyde 3,7 % avec triton 0,1 % en présence de BSA (dilué au 1/100ième). Les cellules sont incubées en présence d'un anticorps monoclonal de souris spécifique de la caspase-14 (BD Biosciences), puis d'un anticorps secondaire, couplé à un marqueur fluorescent. Les cellules sont alors examinées au microscope à Epi-fluorescence (Nikon Eclipse E600 microscope).

### Résultats :

On observe une augmentation de l'intensité lumineuse dans cellules KHN traitées avec le peptide par rapport à la condition contrôle. Cette augmentation de fluorescence est dose-dépendante puisqu'elle est plus importante lorsque la solution de peptide est dosée à 3 % qu'à 1 %. Par conséquent, le peptide SEQ ID n° 2 active l'expression de la caspase-14 dans les KHN.

### Exemple 2 : Etude de l'expression de la caspase-14 par siRNA dans des KHN traités à l'aide du peptide SEQ ID n°2

Afin de quantifier l'efficacité d'un peptide selon l'invention sur la surexpression de la caspase-14 dans une population de KHN, le gène codant pour la caspase-14 a été « éteint » par utilisation de la technique du siRNA.

### Protocole :

Des cellules KHN sont mises en culture en plaque 6 puits jusqu'à une confluence de 60 % puis traitées avec 20 µL de peptide SEQ ID n°2 à 1 %. Ensuite, 100 µL d'un mélange préalablement réalisé contenant le siRNA de la Caspase-14 et l'agent transfectant sont rajoutés délicatement au goutte à goutte, puit par puit. La plaque de culture cellulaire est incubée à 37°C et à 5 % en CO2 pendant 72 heures. Le milieu de culture est renouvelé tous les 2 jours. Quatre conditions sont mises en place :
- condition 1 : contrôle sans siRNA et sans actif peptidique
- condition 2 : cellules transfectées avec le siRNA, sans actif peptidique
- condition 3 : cellules non transfectées mais traitées avec l'actif peptidique
- condition 4 : cellules transfectées avec le siRNA et traitées avec l'actif peptidique

La quantification de l'expression de la Caspase-14 est observée par la technique classique d'immunotransfert (Western Blot) réalisée à l'aide d'un anticorps anti-caspase-14 et selon un protocole classique. Afin d'analyser la compensation apportée par le peptide dans les KHN ayant été transfectés avec le siRNA, la comparaison sera faite par rapport à des KHN non traités et dont le gène n'a pas été éteint par siRNA.

### Résultats :

Entre les conditions 1 et 3, on constate que l'ajout du peptide a entraîné une augmentation de l'expression de la protéine caspase-14 de 20,9 % par rapport au contrôle. Entre les conditions 1 et 2, on constate bien l'effet du siRNA sur l'expression de la protéine caspase-14 : en effet, celle-ci est en baisse de 30,6 %. Par contre; l'ajout du peptide SEQ ID n°2 aux cellules transfectées avec le siRNA permet de restaurer l'expression de la caspase-14, et la baisse due à la présence de siRNA n'est plus que de 14 % par rapport à la condition contrôle.

En conclusion, le peptide SEQ ID n°2 selon l'invention a permis d'augmenter l'expression de la caspase-14 dans les KHN.

### Exemple 3 : Etude de l'expression de la pro/filaggrine dans des biopsies de peau humaine, en présence du peptide SEQ ID n°1

Le but de cette étude est de déterminer l'influence d'un peptide selon l'invention sur la quantité de filaggrine et profilaggrine (pro/filaggrine par la suite) dans des biopsies de peau humaine.

### Protocole :

Des échantillons de peau humaine sont mis en culture à l'interface air/liquide. Dans une première condition, les échantillons sont traités avec le peptide SEQ ID n° à 1 % pendant 24 heures et 72 heures. Dans une seconde condition, les échantillons sont traités avec le même peptide mais concentré à 3 % pendant 24 heures et 72 heures.

Ces échantillons de peau sont ensuite fixés avec du formaldéhyde puis inclus dans de la paraffine. Des coupes de 2 à 3 µm sont alors réalisées. L'immunomarquage est effectué après démasquage des sites spécifiques par incubation dans de la pepsine. L'immunomarquage est alors réalisé à l'aide d'un anticorps monoclonal de souris spécifique de la filaggrine (Tebu Santa Cruz) puis d'un anticorps secondaire, couplé à un marqueur fluorescent. Les coupes de peau sont alors examinées au microscope à Epi-fluorescence (Nikon Eclipse E600 microscope).

### Résultats :

On observe une augmentation du marquage de la pro/filaggrine sur les biopsies traitées à l'aide du peptide selon l'invention. On note que non seulement le marquage est dose-dépendant, mais en outre, celui-ci s'intensifie au cours du temps (entre les conditions 24 heures et 72 heures). On peut donc conclure que le peptide SEQ ID n°1 a permis une augmentation du clivage de la profilaggrine en filaggrine par l'intermédiaire d'une augmentation de l'expression et/ou de l'activité de la caspase-14.

### Exemple 4 : Test comètes sur des cellules KHN

Le test comètes est un test qui permet de quantifier les dommages causés à l'ADN au niveau cellulaire.

### Protocole :

Dans ce but, des cellules KHN sont :
- condition a : mises en culture pendant 24 heures avec le composé peptidique de séquence SEQ ID n° 3 à une concentration de 1 % et de 3 %, puis irradiées avec des rayonnements UVB à raison de 20 mJ/ cm², afin de constater un effet protecteur de l'actif peptidique sur les cellules ;
- condition b : irradiées dans un premier temps avec des rayonnements UVB à raison de 20 mJ/ cm², puis traitées pendant 24 heures par ledit composé peptidique à une concentration de 1 % et de 3 %, afin de constater un effet réparateur de l'actif sur les cellules.

Une condition contrôle est réalisée dans chaque cas sans actif peptidique.

Les cellules sont ensuite trypsinées de leur support, puis centrifugées à 900 tours/min pendant 5 minutes afin de les concentrer et les dénombrer.

Un nombre défini de cellules (25 000 cellules) est ensuite inclut dans un gel d'agarose Low Melting à 0.75 %, puis déposé sur une lame de verre préalablement recouverte d'agarose à 1 %. Les lames sont ensuite immergées dans une solution de lyse pendant 1 heure 30 minutes à 4 °C, puis dans une solution alcaline pendant 20 minutes à 4 °C. Les cellules sont ainsi lysées et l'ADN dénaturé. Les lames sont plongées dans une solution d'électrophorèse avant d'appliquer un champ électrique (20 V - 250 mA). L'ADN ainsi dénaturé est soumis à une migration au sein du gel d'agarose à 4 °C. L'application d'un colorant fluorescent de l'ADN sur les lames (l'iodure de propidium à 2 µg/ ml) permet d'observer au microscope l'ADN apparaissant sous forme de comètes dans le cas où il a été endommagé.

Un logiciel de quantification permet de déterminer le Tail Moment moyen appliqué à chaque condition testée. Ce paramètre renseigne sur le niveau d'endommagement de l'ADN : plus il est élevé, plus les dommages sur l'ADN sont importants.

### Résultats :

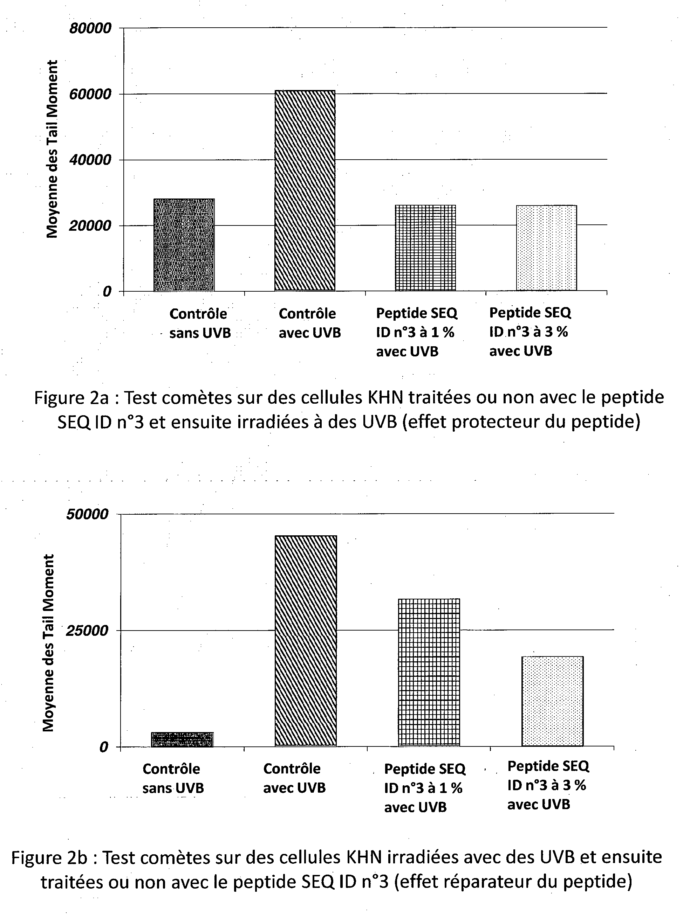

Les résultats sont présentés dans les figures 2a et 2b. Dans la condition a, le Tail Moment diminue de 57 % lorsque le peptide est appliqué en prétraitement (à une concentration de 1 % ou de 3 %), c'est-à-dire que les cellules ont subies moins de dommages que dans la condition contrôle avec rayonnements UVB. Ces résultats confirment bien l'effet protecteur du composé peptidique de séquence SEQ ID n°3 sur les cellules KHN. Par ailleurs, lorsque le peptide est appliqué après irradiation, sans prétraitement (condition b), le Tail Moment diminue de 31 % lorsque le peptide est appliqué à une concentration de 1 %. Le Tail Moment diminue de 58 % lorsque le peptide est appliqué à une concentration de 3 %. Ce test en condition b a permis de mettre en évidence le rôle réparateur du peptide SEQ n°3 sur l'ADN lors d'irradiations UV.

### Exemple 5 : Mise en évidence de l'action réparatrice du peptide SEQ ID n°2 sur des biopsies de peau humaine soumises à des rayonnements UVB

Le but de cette expérience est de mesurer les capacités de réparation d'un composé peptidique selon l'invention après irradiation par rayonnements UVB de biopsies de peau humaine. Le rayonnement UV, et particulièrement UVB, entraine des réactions de dimérisation qui ont lieu aux sites comprenant deux pyrimidines adjacentes (thymidine, cytosine). Plusieurs types de photoproduits sont alors formés, dont les dimères de cyclobutane-pyrimidine ou DCPs. Or, il est connu que lorsqu'une cellule est exposée à un stress génotoxique, son cycle de division est temporairement arrêté pour permettre la réparation de l'ADN et éviter ainsi l'apparition de mutations dans les générations suivantes de cellules. La prolifération cellulaire ne reprend qu'ensuite. Si la fréquence ou la quantité des dommages est trop élevée, ou encore la réparation inefficace, les cellules enclenchent un processus de mort programmée, l'apoptose. Par conséquent, en mesurant par immunomarquage à l'aide d'un anticorps anti-DCPs, la quantité de photoproduits formés, il est possible d'évaluer l'efficacité d'un composé ayant une action réparatrice sur l'ADN.

### Protocole d'immunomarquage des DCPs sur des biopsies de peau soumises à des UVB

Des biopsies de peau humaine sont mises en culture à l'interface air/liquide. Ces biopsies sont soumises à des irradiations par rayonnements UVB à raison de 200 mJ/ cm². Après irradiation, dans une première condition, une solution à 1 % de peptide SEQ ID n°2 est appliquée topiquement sur les biopsies durant 24 heures. Dans une seconde condition, une solution à 3 % de peptide SEQ ID n°2 est appliquée topiquement durant 24 heures. Une condition contrôle est réalisée par application topique d'une solution de PBS après irradiation.

Pour le marquage des dimères cyclobutane-pyrimidine, les biopsies de peau sont incluses dans de la paraffine et des coupes histologiques de 3 µm d'épaisseur sont effectuées. Les lames sont déparaffinées, hydratées puis soumises à un immunomarquage par un anticorps dirigé contre les dimères cyclobutane-pyrimidine (MBL D194-1, mouse monoclonal) puis par un anticorps secondaire adapté (Invitrogen A21202), couplé à un marqueur fluorescent. Les coupes de peau sont alors examinées au microscope à Epi-fluorescence (Nikon Eclipse E 80i microscope).

### Résultats :

En condition contrôle, la fluorescence observée est plus intense lorsque les biopsies ont été soumises aux rayonnements UVB. Dans les deux conditions testées avec post-traitement à l'aide du peptide SEQ ID n°2, on observe une fluorescence beaucoup moins intense que dans la condition contrôle avec UVB.

### Conclusions :

Le peptide SEQ ID n°2 a permis une meilleure et une plus importante réparation des dommages subis par l'ADN, ceci grâce notamment à l'activation de la caspase-14. On observe aussi que cette action de réparation est dose-dépendante puisque celle-ci est plus importante lorsqu'une plus grande quantité d'actif est appliquée.

### Exemple 6 : Composition d'une crème solaire

| Noms commerciaux | Noms INCI | % massique |
|---|---|---|
| PHASE A | | |
| Eau déminéralisée | Aqua (Water) | qsp |
| Pemulen TR1 | Acrylates/C 10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| Noms commerciaux | Noms INCI | % massique |
|---|---|---|
| PHASE D | | |
| Peptide SEQ ID n°3 | | 3 ppm |
| Parfum | Parfum (Fragrance) | qsp |
| Colorant | | qsp |

## Revendications

1. Composé peptidique de formule générale suivante (I) :
R₁ - (AA)n - X₁ - X₂ - Ile - Gln - Ala - Cys - Arg - Gly - X₃ - (AA)ₚ - R₂
Dans laquelle,
X₁ représente un acide aspartique, un acide glutamique ou aucun acide aminé,
X₂ représente une asparagine, une proline, une sérine ou aucun acide aminé,
X₃ représente une asparagine, une arginine, une leucine ou aucun acide aminé,
AA représente un acide aminé quelconque, et n et p sont des nombres entiers compris entre 0 et 2,
R₁ représente la fonction amine primaire de l'acide aminé N-terminal libre,
R₂ représente le groupe hydroxyle de la fonction carboxyle de l'acide aminé C-terminal, substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 6 à 13 résidus d'acides aminés.

2. Composé peptidique selon la revendication 1, **caractérisé en ce qu'**il correspond à une des formules suivantes :
(SEQ ID n°1) Asp - Pro - Ile - Gln - Ala - Cys - Arg - Gly - NH₂
(SEQ ID n°2) Ile - Gln - Ala - Cys - Arg - Gly - NH₂
(SEQ ID n°3) Asn - Arg - Ile - Gln - Ala - Cys - Arg - Gly- NH₂
(SEQ ID n°4) Pro - Ile - Gln - Ala - Cys - Arg - Gly - Phe - NH₂

3. Composé peptidique selon l'une des revendications précédentes **caractérisé en ce qu'**il est utilisé à titre de médicament.

4. Composition cosmétique comprenant à titre de principe actif ledit composé peptidique selon l'une des revendications 1 à 2.

5. Composition cosmétique selon la revendication 4, **caractérisée en ce qu'**elle se présente sous une forme adaptée à l'application par voie topique comprenant un milieu cosmétiquement acceptable.

6. Composition selon l'une des revendications 4 ou 5, **caractérisée en ce que** ledit composé peptidique est présent dans la composition à une concentration comprise entre 0,0005 et 500 ppm environ, et préférentiellement à une concentration comprise entre 0,01 et 5 ppm.

7. Composition selon l'une quelconque des revendications 4 à 6, **caractérisée en ce qu'**elle contient en outre, au moins un autre principe actif favorisant l'action dudit composé peptidique, choisi parmi un agent anti-radicalaire ou antioxydant, ou un agent stimulant la synthèse de macromolécules dermiques, ou encore un agent stimulant le métabolisme énergétique.

8. Utilisation non-thérapeutique d'une composition cosmétique comprenant ledit composé peptidique tel que défini dans l'une des revendications 1 à 2 et un milieu cosmétiquement acceptable pour activer la caspase-14 ainsi que la formation de filaggrine.

9. Composition comprenant ledit composé peptidique tel que défini dans l'une des revendications 1 à 2 pour prévenir et/ou réparer les dommages subis par l'ADN.

10. Composition selon la revendication 9, **caractérisée en ce que** les dommages sont induits par des rayonnements UV.

11. Composition comprenant ledit composé peptidique tel que défini dans l'une des revendications 1 à 2 pour prévenir et/ou traiter les signes cutanés du vieillissement et du photo-vieillissement.

12. Utilisation non-thérapeutique d'une composition cosmétique selon la revendication 8 pour améliorer la fonction barrière de la peau ainsi que l'hydratation de l'épiderme.

13. Composition selon l'une des revendications 9 à 11, **caractérisée en ce qu'**elle est appliquée topiquement sur la peau, pour prévenir et/ou traiter les signes cutanés du vieillissement et du photo-vieillissement, et prévenir et/ou réparer les dommages dus aux rayonnements UV.

14. Composition selon la revendication précédente **caractérisée en ce qu'**elle est appliquée avant une exposition au soleil, en tant que soin avant-soleil afin de prévenir les dommages au niveau de l'ADN.

15. Composition selon la revendication 13 **caractérisée en ce qu'**elle est appliquée après une exposition au soleil, en tant que soin après-soleil afin de réparer les dommages subis au niveau de l'ADN.

16. Procédé de traitement cosmétique non-thérapeutique selon la revendication 8 ou 12 **caractérisé en ce que** la composition est appliquée soit le matin en tant que soin anti-âge de jour, soit le soir au coucher en tant que soin réparateur de nuit

## Patentansprüche

1. Peptidverbindung der folgenden allgemeinen Formel (I):
R₁- (AA) ₙ-X₁-X₂-Ile-Gln-Ala-Cys-Arg-Gly-X₃- (AA) ₚ-R₂
wobei
X₁ für eine Asparaginsäure, eine Glutaminsäure oder keine Aminosäure steht,
X₂ für ein Asparagin, ein Prolin, ein Serin oder keine Aminosäure steht,
X₃ für ein Asparagin, ein Arginin, ein Leucin oder keine Aminosäure steht,
AA für eine beliebige Aminosäure steht, und n und p ganze Zahlen zwischen 0 und 2 sind,
R₁ für die primäre Aminofunktion der freien N-terminalen Aminosäure steht,
R₂ für die Hydroxylgruppe der Carboxylfunktion der C-terminalen Aminosäure steht, ersetzt durch eine Gruppe, die ausgewählt sein kann aus einer Alkylkette mit C₁ bis C₃₀, oder einer NH2-, NHY- oder NYY-Gruppe, wobei Y für eine Alkylkette mit C₁ bis C₄ steht,
wobei die besagte Sequenz der allgemeinen Formel (I) aus 6 bis 13 Aminosäureresten gebildet ist.

2. Peptidverbindung nach Anspruch 1, **dadurch gekennzeichnet, dass** sie einer der folgenden Formeln entspricht:
(SEQ-ID Nr. 1) Asp-Pro-Ile-Gln-Ala-Cys-Arg-Gly-NH₂
(SEQ-ID Nr. 2) Ile-Gln-Ala-Cys-Arg-Gly-NH₂
(SEQ-ID Nr. 3) Asn-Arg-Ile-Gln-Ala-Cys-Arg-Gly-NH₂
(SEQ-ID Nr. 4) Pro-Ile-Gln-Ala-Cys-Arg-Gly-Phe-NH₂

3. Peptidverbindung nach einem der vorstehenden Ansprüche, **dadurch gekennzeichnet, dass** sie als Arzneimittel verwendet wird.

4. Kosmetische Zusammensetzung, die die besagte Peptidverbindung nach einem der Ansprüche 1 bis 2 als Wirkstoff umfasst.

5. Kosmetische Zusammensetzung nach Anspruch 4, **dadurch gekennzeichnet, dass** sie in einer für die topische Anwendung geeigneten Form vorliegt, die ein kosmetisch akzeptables Medium umfasst.

6. Zusammensetzung nach einem der Ansprüche 4 oder 5, **dadurch gekennzeichnet, dass** die besagte Peptidverbindung in einer Konzentration zwischen etwa 0,0005 und 500 ppm, und bevorzugt in einer Konzentration zwischen 0,01 und 5 ppm in der Zusammensetzung vorhanden ist.

7. Zusammensetzung nach einem der Ansprüche 4 bis 6, **dadurch gekennzeichnet, dass** sie des Weiteren mindestens einen anderen Wirkstoff enthält, der die Wirkung der besagten Peptidverbindung begünstigt, ausgewählt aus einem antiradikalen oder antioxidativen Mittel, oder einem Mittel, das die Synthese von dermalen Makromolekülen stimuliert, oder auch einem Mittel, das den Energiestoffwechsel stimuliert.

8. Nichttherapeutische Verwendung einer kosmetischen Zusammensetzung, die die besagte Peptidverbindung wie in einem der Ansprüche 1 bis 2 definiert, und ein kosmetisch akzeptables Medium umfasst, zum Aktivieren der Caspase-14 sowie der Bildung von Filaggrin.

9. Zusammensetzung, die die besagte Peptidverbindung wie in einem der Ansprüche 1 bis 2 definiert umfasst, zum Vorbeugen und/oder Reparieren von an der DNA erlittenen Schäden.

10. Zusammensetzung nach Anspruch 9, **dadurch gekennzeichnet, dass** die Schäden durch UV-Strahlung hervorgerufen werden.

11. Zusammensetzung, die die besagte Peptidverbindung wie in einem der Ansprüche 1 bis 2 definiert umfasst, zum Vorbeugen und/oder Behandeln der Anzeichen der Hautalterung und der Lichtalterung.

12. Nichttherapeutische Verwendung einer kosmetischen Zusammensetzung nach Anspruch 8 zum Verbessern der Barrierefunktion der Haut sowie der Hydratation der Epidermis.

13. Zusammensetzung nach einem der Ansprüche 9 bis 11, **dadurch gekennzeichnet, dass** sie topisch auf der Haut aufgetragen wird, zum Vorbeugen und/oder Behandeln der Anzeichen der Hautalterung und der Lichtalterung, und Vorbeugen und/oder Reparieren der durch UV-Strahlung bedingten Schäden.

14. Zusammensetzung nach dem vorstehenden Anspruch, **dadurch gekennzeichnet, dass** sie vor einer Exposition gegenüber Sonnenlicht aufgetragen wird als sonnenvorbereitende Pflege, um Schäden an der DNA vorzubeugen.

15. Zusammensetzung nach Anspruch 13, **dadurch gekennzeichnet, dass** sie nach einer Exposition gegenüber Sonnenlicht aufgetragen wird als After-Sun-Pflege, um die an der DNA erlittenen Schäden zu reparieren.

16. Nichttherapeutisches kosmetisches Behandlungsverfahren nach Anspruch 8 oder 12, **dadurch gekennzeichnet, dass** die Zusammensetzung entweder morgens als Anti-Aging-Tagespflege, oder abends vor dem Zubettgehen als reparierende Nachtpflege aufgetragen wird.

## Claims

1. Peptide compound having the following general formula (I):
R₁- (AA) ₙ-X₁-X₂-Ile-Gln-Ala-Cys-Arg-Gly-X₃- (AA) ₚ-R₂
Wherein,
X₁ represents an aspartic acid, a glutamic acid or no amino acid,
X₂ represents an asparagine, a proline, a serine or no amino acid,
X₃ represents an asparagine, an arginine, a leucine or no amino acid,
AA represents any amino acid, and n and p are integers between 0 and 2,
R₁ represents the primary amine function of the free N-terminal amino acid,
R₂ represents the hydroxyl group of the carboxyl function of the C-terminal amino acid, substituted by a group that may be chosen from among a C₁ to C₃₀ alkyl chain, or an NH2, NHY or NYY group where Y represents a C₁ to C₄ alkyl chain,
said sequence having the general formula (I) consisting of 6 to 13 amino acid residues.

2. Peptide compound according to claim 1, **characterised in that** it corresponds to one of the following formulae:
(SEQ ID No. 1) Asp-Pro-Ile-Gln-Ala-Cys-Arg-Gly-NH₂
(SEQ ID No. 2) Ile-Gln-Ala-Cys-Arg-Gly-NH₂
(SEQ ID No. 3) Asn-Arg-Ile-Gln-Ala-Cys-Arg-Gly-NH₂
(SEQ ID No. 4) Pro-Ile-Gln-Ala-Cys-Arg-Gly-Phe-NH₂

3. Peptide compound according to any one of the preceding claims **characterised in that** it is used as a medicine.

4. Cosmetic composition comprising, as an active ingredient, said peptide compound according to any one of claims 1 or 2.

5. Cosmetic composition according to claim 4, **characterised in that** it is presented in a form suitable for topical application comprising a cosmetically acceptable medium.

6. Composition according to any one of claims 4 or 5, **characterised in that** said peptide compound is present in the composition at a concentration between approximately 0.0005 and 500 ppm, and preferentially at a concentration between 0.01 and 5 ppm.

7. Composition according to any one of claims 4 to 6, **characterised in that** it further contains at least one further active ingredient promoting the action of said peptide compound, chosen from among an anti-radical or anti-oxidant agent, or an agent stimulating the synthesis of dermal macromolecules, or else an agent stimulating the energy metabolism.

8. Non-therapeutic use of a cosmetic composition comprising said peptide compound as defined in any one of claims 1 to 2 and a cosmetically acceptable medium for activating caspase-14 as well as filaggrin formation.

9. Composition comprising said peptide compound as defined in any one of claims 1 to 2 for preventing and/or treating damage sustained by DNA.

10. Composition according to claim 9, **characterised in that** the damage is caused by UV radiation.

11. Composition comprising said peptide compound as defined in any one of claims 1 to 2 for preventing and/or treating the skin signs of ageing and photo-ageing.

12. Non-therapeutic use of a cosmetic composition according to claim 8 for enhancing the skin barrier function as well as epidermal hydration.

13. Composition according to any one of claims 9 to 11, **characterised in that** it is applied topically onto the skin, for preventing and/or treating the skin signs of ageing and photo-ageing, and preventing and/or repairing damage caused by UV radiation.

14. Composition according to the preceding claim **characterised in that** it is applied before exposure to the sun, as a pre-sun treatment in order to prevent damage to DNA.

15. Composition according to claim 13 **characterised in that** it is applied after exposure to the sun, as an after-sun treatment in order to prevent damage to DNA.

16. Non-therapeutic cosmetic treatment method according to claim 8 or 12 **characterised in that** the composition is applied either in the morning as a day anti-ageing treatment, or in the evening at bedtime as a night repair treatment.


### SEQUENCE LISTING

<110> ISP Investments Inc
<120> NOUVEAUX PEPTIDES ACTIVATEURS DE LA CASPASE-14 ET COMPOSITIONS LES COMPRENANT
<130> Bv PCT 10-137
<150> 10 00463
   <151> 2010-02-05
<160> 4
<170> PatentIn version 3.3
<210> 1
   <211> 8
   <212> PRT
   <213> Artificial
<220>
   <223> Synthetic peptide
<220>
   <221> MOD_RES
   <222> (8)..(8)
   <223> AMIDATION
<400> 1
   Asp Pro Ile Gln Ala Cys Arg Gly
   1 5
<210> 2
   <211> 6
   <212> PRT
   <213> Artificial
<220>
   <223> Synthetic peptide
<220>
   <221> MOD_RES
   <222> (6)..(6)
   <223> AMIDATION
<400> 2
   Ile Gln Ala Cys Arg Gly
   1 5
<210> 3
   <211> 8
   <212> PRT
   <213> Artificial
<220>
   <223> Synthetic peptide
<220>
   <221> MOD_RES
   <222> (8)..(8)
   <223> AMIDATION
<400> 3
   Asn Arg Ile Gln Ala Cys Arg Gly
   1 5
<210> 4
   <211> 8
   <212> PRT
   <213> Artificial
<220>
   <223> Synthetic peptide
<220>
   <221> MOD_RES
   <222> (8)..(8)
   <223> AMIDATION
<400> 4
   Pro Ile Gln Ala Cys Arg Gly Phe
   1               5